Europäisches Patentamt

European Patent Office

Office européen des brevets

(11) Publication number: **0 674 897 A2**

# EUROPEAN PATENT APPLICATION

(21) Application number: **95104808.1**

(51) Int. Cl.6: **A61K 7/06**

(22) Date of filing: **31.03.95**

(30) Priority: **31.03.94 JP 85461/94**
**25.08.94 JP 222690/94**

(43) Date of publication of application:
**04.10.95 Bulletin 95/40**

(84) Designated Contracting States:
**CH DE ES FR GB IT LI NL SE**

(71) Applicant: **SHISEIDO COMPANY LIMITED**
**5-5 Ginza 7-chome**
**Chuo-ku**
**Tokyo (JP)**

(72) Inventor: **Hanzawa, Chika, c/o Shiseido**
**Research Center (1)**
**1050, Nippa-cho,**
**Kohoku-ku**
**Yokohama-shi,**
**Kanagawa-ken (JP)**
Inventor: **Matsubuchi, Eriko, c/o Shiseido**
**Research Center(1)**
**1050, Nippa-cho,**
**Kohoku-ku**
**Yokohama-shi,**
**Kanagawa-ken (JP)**
Inventor: **Nishi, Toyoyuki**
**35, Nishimachi**
**Kameoka-shi,**
**Kyoto-fu (JP)**

(74) Representative: **Kraus, Walter, Dr. et al**
**Patentanwälte Kraus, Weisert & Partner**
**Thomas-Wimmer-Ring 15**
**D-80539 München (DE)**

(54) **Hair composition.**

(57) A composition for the head comprising an effective amount of an extract of a plant of the family Berberidaceae Juss., a plant of the family Flacourtiaceae DC. or a plant of the family Euphorbiaceae Juss., and auxiliaries. The composition for the head is effective for prevention of dandruff and hair loss, and hair growth promotion (or hair revitalization promotion).

EP 0 674 897 A2

## BACKGROUND OF THE INVENTION

### 1. Field of the Invention

This invention relates to a composition for the head containing as an effective ingredient an extract derived from a plant, particularly to a composition which exerts effects such as hair revitalization, hair loss prevention and hair growth induction. More specifically, as the extract of a plant as the effective ingredient is used an extract of the genus Podophyllum L. of the family Berberidaceae Juss. the genus Casearia Jacq. of the family Flacourtiaceae DC. and the genus Euphorbia L. of the family Euphorbiaceae Juss.

### 2. Description of the Prior Art

Heretofore, activation of male hormones in organs such as hair root and sebaceus glands; lowering of bloodstream volume to hair follicles; hypersteatosis; anomaly of scalps due to formation of peroxides, etc.; etc. have been considered to be causes of baldness and hair loss. Thus, compounds having an action to remove or reduce these causes are generally incorporated in usual hair tonics.

As such compounds, for example vitamins such as vitamin B and vitamin E, amino acids such as serine and methionine, vasodilators such as Swertia japonica extract and acetylcholine derivatives, anti-inflammatory agents such as Lithospermi Radix extract and hinokitiol, female hormones such as estradiol, skin function promotors such as cepharanthine, etc. are incorporated, and used for prophylaxis and treatment of alopecia. Further, some proposals are made on utilization of specific plant ingredients.

However, although various attempts have been made as stated above, the hair-tonic actions such as hair loss prevention effect and hair growth inducing effect of usual hair tonics are not always sufficient. The reason is considered, probably, to be that there are various causes of hair loss and the mechanism of hair growth is very complicated. Therefore, there still exists a demand for obtaining compounds or compositions more effective for hair loss prevention, hair growth, etc. than usual ones, and excellent in safety.

Thus, the object of this invention lies in providing a composition for the head effective for hair loss prevention, hair growth, etc.

## SUMMARY OF THE INVENTION

The present inventors had thereto vigorously studied on effectiveness of various plant ingredients for hair loss prevention, hair growth, etc., found that extracts derived, particularly from plants of the genus Podophyllum L. of the family Berberidaceae Juss., plants of the genus Casearia Jacq. of the family Flacourtiaceae DC. and plants of the genus Euphorbia L. of the family Euphorbiaceae Juss. exhibit the above effectiveness and are excellent in safety, and completed this invention.

Thus, according to this invention is provided a composition comprising an extract derived from a plant, in an amount effective for hair revitalization, hair loss prevention or hair growth induction, and pharmaceutically acceptable auxiliaries, said plant being one selected from plants of the genus Podophyllum L. of the family Berberidaceae Juss., plants of the genus Casearia Jacq. of the family Flacourtiaceae DC. and plants of the genus Euphorbia L. of the family Euphorbiaceae Juss.

According to this invention is further provided a method for revitalizing hair, preventing hair loss or inducing hair growth on a mammal, which comprises applying the above composition to the scalp or hair.

According to this invention is further provided use of an extract derived from the above plant for preparing a composition for hair revitalization, hair loss prevention or hair growth induction of mammals.

## DETAILED DESCRIPTION OF THE INVENTION

The composition of this invention can contain as an indispensable ingredient an effective amount of an extract derived from at least one of plants of the genus Podophyllum L. of the family Berberidaceae Juss. Specific examples of plants of the genus Podophyllum L. include Podophyllum hexandrum Royle (hereafter, the name of this plant is abbreviated as "P. hex."), Podophyllum pleianthum Hance (hereafter, the name of this plant is abbreviated as "P. ple."), Podophyllum versipella Hance (hereafter, the name of this plant is abbreviated as "P. ver."), Podophyllum emodi Wall (hereafter, the name of this plant is abbreviated as "P. emo."), and Podophyllum peltatum L. (hereafter, the name of this plant is abbreviated as "P. pel.").

The composition of this invention can also contain as an indispensable ingredient an extract derived from at least one of plants of the genus Casearia Jacq. of the family Flacourtiaceae DC., in place of the above extract or together with the extract. Specific examples of plants of the genus Casearia Jacq. include Casearia merrilli Hayatae (hereafter, the name of this plant is abbreviated as "C. mer."), Casearia cauliflora Volkens (hereafter, the name of this plant is abbreviated as "C. cau."), Casearia esculenta Roxb. (hereafter, the name of this plant is abbreviated as "C. esc."), Casearia graveolens Daltz. (hereafter, the name of this plant is abbreviated as "C. gra."), Casearia ovata Willd. (hereafter, the name of this plant is abbreviated as "C. ova."), and Casearia grewiaefolia Miq. (hereafter, the name of this plant is abbreviated as "C. gre.").

The composition of this invention can also contain as an indispensable ingredient an extract derived from at least one of plants of the genus Euphorbia L. of the family Euphorbiaceae Juss., in place of the above both extracts or together with one or both of these extracts. Specific examples of plants of the genus Euphorbia L. include Euphorbia helioscopia L. (hereafter, the name of this plant is abbreviated as "E. hel."), Euphorbia lathyris L. (hereafter, the name of this plant is abbreviated as "E. lat."), Euphorbia neriifolia L. (hereafter, the name of this plant is abbreviated as "E. ner."), Euphorbia pekinensis Rupr.(hereafter, the name of this plant is abbreviated as "E. pek."), Euphorbia supina Rafin (hereafter, the name of this plant is abbreviated as "E. sup.") and Euphorbia trigona Haw. (hereafter, the name of this plant is abbreviated as "E. tri.").

As stated above, the composition of this invention can contain one or more plant extracts derived from different families or genera, or can contain plant extracts derived from one or more species belonging to the same genus.

Parts constituting a plant body, used in this invention, are not particularly limited, and leaves, bark, wood, branches and roots (when each is applicable) are mentioned. Extraction of effective ingredients from these plant body - constituting parts can be carried out, if necessary after these constituting parts being dried and powdered, using, as an extracting solvent, one or any mixture of water, lower alkyl alcohols such as methanol, ethanol, propanol and isopropanol, ketones such as acetone and methyl ethyl ketone, esters such as ethyl acetate and butyl acetate, glycols such as ethylene glycol and 1,3-butylene glycol. It is sometimes recommended to carry out the extraction under heating.

The thus obtained extracts can be used, in accordance with dosage forms of compositions for heads containing these extracts, as they are, as concentrates after being concentrated, or as dry matter or viscous matter (hereafter, sometimes referred to as extract) after the extracting solvents being distilled off.

The content of the above extract in the composition for the bead of this invention is not limited because the optimum amount varies depending on its dosage form, but the content per the composition is generally adjusted to 0.005 to 20 weight %, preferably 0.01 to 10 weight %. When the content is under 0.005 weight %, the effects of this invention is not adequately displayed, and the content above 20 weight % is not preferable because of disadvantage on compounding.

The composition for the head of this invention can, if necessary, contain various auxiliaries generally used in cosmetics, quasi-drug, drugs, and so on, in such arrange that the effects of this invention is not spoiled, besides the above indispensable ingredient. These auxiliaries include, for example, oils such as polyoxyethylene (8 mols) and glyceryl monooleate, bloodstream promotors such as nicotinamide, benzyl nicotinate, vitamin E acetate and Swertia japonica extract; testosterone-$\alpha$-reductase inhibitors such as glycyrrhetinic acid and hinokitiol; hormones such as ethynylestradiol; hair root activators such as vitamin H and pantothenyl ethyl ether; nonionic surface active agents such as sorbitan monolaurate, sorbitan monopalmitate, sorbitan sesquioleate, sorbitan trioleate, polyoxyethylenesorbitan monolaurate, polyoxyethylenesorbitan monostearate, polyethylene glycol monooleate, polyoxyethylene alkyl ethers, polyglycol diesters, lauryl diethanolamide and fatty acid isopropanolamides; cationic surface active agents such as stearyltrimethylammonium chloride and benzalkonium chloride; anionic surface active agents such as sodium palmitate, sodium lauryl sulfate, potassium lauryl sulfate, triethanolamine alkyl sulfates, sulfonated oils, linear dodecylbenzene sulfate and polyoxyethylene hardened castor oil maleate; semi-polar surface active agents such as lauryldimethylamine oxide and oleicdimethylamine oxide; amphoteric surface active agents; humectants; thickners; antiseptic agents; antioxidants; perfumes; colorants; etc.

The composition for the head of this invention is not limited on its dosage forms, and can take dosage forms such as, for example, tonics, hair creams, shampoos, scalp treatments, rinses, etc., and preparation thereof can be made according to processes known per se. When administered to the scalps or hair of mammals including human beings, the composition of this invention is capable of hair revitalization, hair loss prevention and hair growth induction. When percutaneous administration of the composition to the head is taken as an example, the dose of the composition in terms of the weight of the plant extract for attaining such effects is, in the case of an adult, 0.01 to 100 mg, preferably 0.1 to 10 mg per kg of the body weight and per once a day. This dose can be administered once or in several divisions per day.

In these dosage forms, the effects of prevention of dandruff and/or hair loss of subjects or hair growth induction of them are observed.

This invention is specifically, more detailedly described below, but the technical scope of this invention is not limited thereby. Mixing amounts in the following description mean weight % per the weight of the whole preparation, unless otherwise described.

Preparation of extracts

Examples 1 to 6: Preparation of extracts of plants of the genus Podophyllum L. of the family Berberidaceae Juss.

Powders of the various plant parts of the various plants of the genus Podophyllum L. of the family Berberidaceae Juss. shown in the following Table 1 were twice extracted at room temperature (15 to 25°C) using extracting solvents and their use amounts, and extraction times shown in Table 1, respectively. Both extracts from each extraction were combined, and concentrated under reduced pressure to dryness to give an extract as shown in Table 1.

## Table 1

| Example No. | Plant | Site | Amount of used sample | Extractant | Amount of used extractant first | second | Extraction time | Amount of obtained extract | Yield |
|---|---|---|---|---|---|---|---|---|---|
| 1 | P.pel. | Root | 225 g | Methanol | 1 ℓ | 1 ℓ | 24 hours x twice | 22 g | 9.8% |
| 2 | P.hex. | Part on the ground | 1150 g | Ethanol | 8 ℓ | 8 ℓ | 24 hours x twice | 195 g | 17.0% |
| 3 | P.pel. | Part on the ground | 2300 g | Ethyl acetate | 10 ℓ | 10 ℓ | 24 hours x twice | 92 g | 4.0% |
| 4 | P.ver. | Root | 2500 g | Methanol | 10 ℓ | 10 ℓ | 24 hours x twice | 220 g | 8.8% |
| 5 | P.emo. | Whole grass | 340 g | Methanol | 2 ℓ | 2 ℓ | 24 hours x twice | 50 g | 14.7% |
| 6 | P.pel. | Part on the ground | 115 g | Ethanol | 1 ℓ | 1 ℓ | 24 hours x twice | 24 g | 20.9% |

EP 0 674 897 A2

Examples 7 to 18: Preparation of extracts of plants of the genus Casearia Jacq. of the family Flacourtiaceae DC.

Powders of the various plant parts of the various plants of the genus Casearia Jacq. of the family Flacourtieceae DC., in place of the plants of the genus Podophyllum L., were extracted under the respective conditions described in Table 2. The results of the extraction are shown together in Table 2.

EP 0 674 897 A2

Table 2

| Example No. | Plant | Site | Amount of used sample | Extractant | Amount of used extractant | | Extraction time | Amount of obtained extract | Yield |
|---|---|---|---|---|---|---|---|---|---|
| | | | | | first | Second | | | |
| 7 | C.gre. | Root | 1150 g | Methanol | 10 ℓ | 5 ℓ | 24 hours x twice | 42 g | 3.7% |
| 8 | C.gre. | Root | 1200 g | Acetone | 10 ℓ | 5 ℓ | 24 hours x twice | 31 g | 2.6% |
| 9 | C.gre. | Root | 1150 g | Ethanol | 10 ℓ | 5 ℓ | 24 hours x twice | 30 g | 2.6% |
| 10 | C.gre. | Root | 1080 g | Ethyl acetate | 10 ℓ | 5 ℓ | 24 hours x twice | 25 g | 2.3% |
| 11 | C.mer. | Root | 980 g | Ethanol | 10 ℓ | 5 ℓ | 24 hours x twice | 32 g | 3.9% |
| 12 | C.cau. | Leaf | 465 g | Ethanol | 2 ℓ | 2 ℓ | 24 hours x twice | 49 g | 10.5% |
| 13 | C.esc. | Leaf | 2500 g | Ethyl acetate | 10 ℓ | 10 ℓ | 24 hours x twice | 68 g | 2.7% |
| 14 | C.gra. | Bark | 1500 g | Methanol | 10 ℓ | 10 ℓ | 24 hours x twice | 146 g | 10.1% |
| 15 | C.ova. | Bark | 1450 g | Ethanol | 10 ℓ | 10 ℓ | 24 hours x twice | 132 g | 9.1% |
| 16 | C.gre. | Leaf | 560 g | Methanol | 2 ℓ | 2 ℓ | 24 hours x twice | 63 g | 11.3% |
| 17 | C.gre. | Bark | 1220 g | Ethyl acetate | 10 ℓ | 10 ℓ | 24 hours x twice | 38 g | 3.1% |
| 18 | C.gre. | Seed | 1340 g | Methanol | 10 ℓ | 10 ℓ | 24 hours x twice | 166 g | 12.4% |

Examples 19 to 24: Preparation of extracts of plants of the genus Euphorbia L. of the family Euphorbiaceae Juss.

Powders of the various plant parts of the various plants of the genus Euphorbia L. of the family Euphorbiaceae Juss., in place of the plants of the genus Podophyllum L., were extracted under the respective conditions described in Table 3. The results of the extraction are shown together in Table 3.

Table 3

| Example No. | Plant | Site | Amount of used sample | Extractant | Amount of used extractant | | Extraction time | Amount of obtained extract | Yield |
|---|---|---|---|---|---|---|---|---|---|
| | | | | | first | second | | | |
| 19 | E.tri. | Wood | 1375 g | Methanol | 7 ℓ | 7 ℓ | 24 hours x twice | 44 g | 3.2% |
| 20 | E.hel. | Part on the ground | 1230 g | Eethanol | 10 ℓ | 10 ℓ | 24 hours x twice | 37 g | 3.0% |
| 21 | E.lat. | Whole grass | 760 g | Methanol | 3 ℓ | 2 ℓ | 24 hours x twice | 69 g | 9.1% |
| 22 | E.ner. | Part on the ground | 2500 g | Ethanol | 10 ℓ | 10 ℓ | 24 hours x twice | 79 g | 3.2% |
| 23 | E.pex. | Part on the ground | 195 g | Methanol | 1 ℓ | 1 ℓ | 24 hours x twice | 19 g | 9.7% |
| 24 | E.sup. | Bark | 190 g | Methanol | 1 ℓ | 1 ℓ | 24 hours x twice | 14 g | 7.4% |

Preparation example

Examples 25 to 38

Each effective ingredient (extract) shown in the following Table 4 was dissolved (or suspended), respectively, in 95% ethanol (A), and deionized water (B) and, in some cases, further hardened castor oil-ethylene oxide (40 mols) adduct (C) were added. The mixture was stirred to give a solution, and deionized water (B) was added to give a liquid composition. The respective compounding rates were as shown in Table 4.

Table 4

| Example No. | Effective ingredient (extract) | | | | | A | B | C |
|---|---|---|---|---|---|---|---|---|
| | P.hex. (part on the ground) | P.ple. (part on the ground) | P.ver. (root) | P.emo. (whole grass) | P.pel. (part on the ground) | | | |
| 25 | 1.8 | 0 | 0 | 0.2 | 0 | 60.0 | 36.0 | 2.0 |
| 26 | 0 | 1.0 | 1.0 | 0 | 0 | 60.0 | 36.0 | 2.0 |
| 27 | 0 | 0 | 1.5 | 0 | 0.5 | 60.0 | 36.0 | 2.0 |
| 28 | 0.3 | 0.2 | 0 | 0.5 | 1.0 | 60.0 | 36.0 | 2.0 |
| 29 | 0 | 0 | 0 | 0 | 2.0 | 60.0 | 36.0 | 2.0 |
| 30 | 2.0 | 0 | 0 | 0 | 0 | 60.0 | 38.0 | 0 |
| 31 | 0 | 2.0 | 0 | 0 | 0 | 60.0 | 38.0 | 0 |
| 32 | 0 | 0 | 2.0 | 0 | 0 | 60.0 | 38.0 | 0 |
| 33 | 0 | 0 | 0 | 2.0 | 0 | 60.0 | 38.0 | 0 |
| 34 | 0 | 0 | 0 | 0 | 2.0 | 60.0 | 38.0 | 0 |
| 35 | 0 | 0 | 1.0 | 0 | 1.0 | 60.0 | 38.0 | 0 |
| 36 | 0.1 | 0.3 | 0.2 | 0.1 | 0.3 | 60.0 | 39.0 | 0 |
| 37 (comparison) | 0 | 0 | 0 | 0 | 0 | 60.0 | 38.0 | 2.0 |
| 38 (comparison) | 0 | 0 | 0 | 0 | 0 | 60.0 | 40.0 | 0 |

Examples 39 to 52

Each effective ingredient (extract) shown in the following Table 5 was dissolved (or suspended), respectively, in 95% ethanol (A), and deionized water (B) and, in some cases, further hardened castor oil-ethylene oxide (40 mols) adduct (C) were added. The mixture was stirred to give a solution, and deionized water (B) was added to give a liquid composition. The respective mixing rates were as shown in Table 5.

Table 5

| Exam-ple No. | Effective ingredient (extract) | | | | | | | | | | | |
|---|---|---|---|---|---|---|---|---|---|---|---|---|
| | C.gre. (Root) | C.gre. (Leaf) | C.gre. (Bark) | C.gre. (Seed) | C.gra. (Bark) | C.esc. (Leaf) | C.mer. (Root) | C.ova. (Bark) | C.cau (Leaf) | A | B | C |
| 39 | 2.0 | 0 | 0 | 0 | 0 | 0 | 0 | 0 | 0 | 60.0 | 38.0 | 0 |
| 40 | 0 | 0 | 0 | 0 | 0 | 0 | 2.0 | 0 | 0 | 60.0 | 36.0 | 2.0 |
| 41 | 0.5 | 0 | 0 | 0 | 0.1 | 0.1 | 0.4 | 0 | 0 | 60.0 | 36.0 | 2.0 |
| 42 | 2.0 | 0 | 0 | 0 | 0 | 0 | 0 | 0 | 0 | 60.0 | 38.0 | 0 |
| 43 | 0 | 0 | 0 | 0 | 2.0 | 0 | 0 | 0 | 0 | 60.0 | 38.0 | 0 |
| 44 | 0 | 0 | 0 | 0 | 0 | 2.0 | 0 | 0 | 0 | 60.0 | 38.0 | 0 |
| 45 | 0 | 0 | 0 | 0 | 0 | 0 | 2.0 | 0 | 0 | 60.0 | 38.0 | 0 |
| 46 | 0.6 | 0 | 0 | 0 | 0.2 | 0.2 | 0.6 | 0.2 | 0.2 | 60.0 | 38.0 | 2.0 |
| 47 | 2.0 | 0 | 0 | 0 | 0 | 0 | 0 | 0 | 0 | 60.0 | 36.0 | 2.0 |
| 48 | 2.0 | 0 | 0 | 0 | 0 | 0 | 0 | 0 | 0 | 60.0 | 38.0 | 0 |
| 49 | 2.0 | 0 | 0 | 0 | 0 | 0 | 0 | 0 | 0 | 60.0 | 38.0 | 0 |
| 50 | 0 | 2.0 | 2.0 | 0 | 0 | 0 | 0 | 0 | 0 | 60.0 | 38.0 | 0 |
| 51 | 0 | 0 | 2.0 | 0 | 0 | 0 | 0 | 0 | 0 | 60.0 | 38.0 | 0 |
| 52 | 0 | 0 | 0 | 2.0 | 0 | 0 | 0 | 0 | 0 | 60.0 | 38.0 | 2.0 |
| 53 (comparison) | 0 | 0 | 0 | 0 | 0 | 0 | 0 | 0 | 0 | 60.0 | 38.0 | 0 |
| 54 (comparison) | 0 | 0 | 0 | 0 | 0 | 0 | 0 | 0 | 0 | 60.0 | 40.0 | 0 |

Examples 39, 46 and 50-52 (Methanol extraction), Example 47 (Acetone extraction), Example 48 (Ethanol extraction), Example 49 (Ethyl acetate extraction)

Examples 55 to 59

Each effective ingredient (extract) shown in the following Table 6 was dissolved (or suspended), respectively, in 75% ethanol, and hardened castor oil-ethylene oxide (40 mols) adduct was added. The mixture was stirred to give a solution, and 75% ethanol was added to make the total mixing weight % to 100%, whereby a liquid composition was obtained. The respective compounding rates of the effective ingredients were as shown in Table 6.

Table 6

| Example No. | | Effective ingredient (extract) | | | |
|---|---|---|---|---|---|
| | E.tri. (Wood) | E.hel. (part on the ground) | E.lat. (whole grass) | E.ner. (part on the ground) | E.pek. (part on the ground) |
| 55 | 1.0 | 0 | 0 | 0 | 0 |
| 56 | 0 | 0.5 | 0 | 0 | 0 |
| 57 | 0 | 0 | 3 | 0 | 0 |
| 58 | 0 | 0.7 | 0 | 0.05 | 0.1 |
| 59 *(comparison) | 0 | 0 | 0 | 0 | 0 |

* Comparison shows the result when only 75% ethanol was used.

Hair revitalization test

Experiment was carried out according to the method of Ogawa et al. (Normal and Abnormal Epidermal Differentiation, edited by M. Seiji and I.A. Berstein, published by Todai Shuppan-kai), using male C3H mice (60 days after birth). Hair on the back of each mouse was shaved into a size of about 2 x 4cm. Samples (the above preparations) were applied once a day to the mice (every day) respectively, and change of the area of the part where hair regeneration began was checked on each mouse, and thereby the speeds of hair regeneration were compared. 10 mice were used for each sample, and the average value of the areas was calculated on each sample. Effect was expressed by (i) days needed for attaining a hair revitalization rate of 50% on each sample, or by (ii) the area, given by a percentage, of hair regenerated 25 days thereafter.

The respective results are shown together in Tables 7 to 9.

Table 7

| Results of hair revitalization test in mice (i) | |
|---|---|
| Sample (Preparation example) | Days required for 50% hair growth |
| Application was not made | 32 (days) |
| Example 25 | 24 |
| Example 26 | 22 |
| Example 27 | 23 |
| Example 28 | 24 |
| Example 29 | 22 |
| Example 30 | 21 |
| Example 31 | 24 |
| Example 31 | 24 |
| Example 33 | 22 |
| Example 34 | 20 |
| Example 35 | 23 |
| Example 36 | 26 |
| Example 37 (comparison) | 32 |
| Example 38 (comparison) | 33 |

Table 8

| Results of hair revitalization test in mice (i) | |
| --- | --- |
| Sample (Preparation example) | Days required for 50% hair growth |
| Application was not made | 31 (days) |
| Example 39 | 18 |
| Example 40 | 22 |
| Example 41 | 25 |
| Example 42 | 19 |
| Example 43 | 23 |
| Example 44 | 24 |
| Example 45 | 22 |
| Example 46 | 22 |
| Example 47 | 18 |
| Example 48 | 19 |
| Example 49 | 17 |
| Example 50 | 23 |
| Example 51 | 20 |
| Example 52 | 24 |
| Example 53 (comparison) | 31 |
| Example 54 (comparison) | 31 |

Table 9

| Results of hair revitalization test in mice (ii) | |
| --- | --- |
| Sample (Preparation example) | Area of revitalized hair (%) |
| Example 55 | 83.5 (%) |
| Example 56 | 68.6 |
| Example 57 | 95.0 |
| Example 58 | 65.1 |
| Example 59 (comparison) | 5.0 |

As apparent from the above results, a significant effect on regeneration of hair was ascertained in each preparation example (this invention).

Trichogram test

Hair roots of hairs pulled out before and after use of compositions for the head were observed by a microscope, the numbers of telogen hair roots distinguished by the form of the hair roots were counted, and the hair-tonic actions of the compositions were compared by increase or decrease of their rates. Telogen hair root means the hair root of a hair whose growth stopped, and it is recognized that persons who complain of hair loss have a higher rate of these telogen hair roots than normal persons.

Each of the samples obtained in the above preparation examples was applied to the scalps of 20 or 10 male subjects twice a day, in an amount of 2 ml per application, successively for 6 months. 100 hairs were pulled out per subject immediately before the application and immediately before completion of 6 months application, respectively, and their hair roots were observed. The results are shown in Tables 10 and 11.

13

## Table 10 Results of trichogram test (20 subjects)

| Sample (preparation example) | Change of [rate of hair roots of resting period after application] to [rate of hair roots of resting period before application] Unit [person] | | | | | Judgement of effect |
|---|---|---|---|---|---|---|
| | 30% or more decreased | 15-30% decreased | ±15% | 15-30% increased | 30% or more increased | |
| Example 25 | 2 | 11 | 6 | 1 | 0 | effective |
| Example 26 | 1 | 13 | 6 | 0 | 0 | effective |
| Example 27 | 2 | 12 | 5 | 1 | 0 | effective |
| Example 37 (comparison) | 0 | 1 | 12 | 7 | 0 | noneffective |

EP 0 674 897 A2

Table 11 Results of trichogram test (10 subjects)

| Sample (preparation example) | Change of [rate of hair roots of resting period after application] to [rate of hair roots of resting period before application] Unit [person] | | | | | Judgement of effect |
|---|---|---|---|---|---|---|
| | 30% or more decreased | 15-30% decreased | ±15% | 15-30% increased | 30% or more increased | |
| Example 39 | 1 | 6 | 2 | 1 | 0 | effective |
| Example 40 | 1 | 5 | 3 | 1 | 0 | effective |
| Example 41 | 1 | 5 | 2 | 2 | 0 | effective |
| Example 47 | 1 | 6 | 1 | 2 | 0 | effective |
| Example 53 (comparison) | 0 | 1 | 5 | 4 | 0 | noneffective |

From the above results, it is understood that the compositions of this invention have an action to remarkably reduce the number of telogen hair roots. This action is also observed likewise in other compositions of this invention.

It is ascertained through this test that the scalps are not badly influenced at all by the compositions of this invention, as is the case in the preparation of the comparative example.

Actual use test on prevention of dandruff and hair loss, and promotion of hair growth

For carrying out the captioned test using a hair tonic, a hair tonic of the following composition was prepared (Preparation example 60) using the dried methanol extract (extract) of E. tri. (wood) obtained in Example 16 and the dried methanol extract (extract) of E. pek. (part on ground) obtained in Example 20.

| Composition | (weight / volume %) |
|---|---|
| Extract of Example 19 | 0.5 |
| Extract of Example 23 | 0.1 |
| Propylene glycol | 5.0 |
| Sodium hyaluronata | 0.01 |
| 75% Ethanol (adjusted to 100%) | |

The above hair tonic was administered 1 to 2 times per day, in 1 to 3 ml portions, over the period of 4 months, to 15 men (age 25 to 55) exhibiting symptoms such as dandruff and hair loss, and results of the following Table 12 were obtained. As apparent from Table 12, this hair tonic shows excellent effects of hair growth promotion and prevention of dandruff and hair loss.

The effects of the following Table 12 are expressed according to the following criteria.

(1) Test on dandruff prevention effect

"noneffective" means a case where no improvement was bound in spite of treatment.

"effective" means a case where occurrence of dandruff was reduced.

"remarkably effective" means a case where occurrence of dandruff was stopped.

(2) Test on hair growth effect

"noneffective" means a case where no improvement was found in spite of treatment.

"effective" means a case where growth of hair was observed in more than 2/3 parts of the area of hair loss.

"remarkably effective" means a case where growth of hair was observed on the whole of the area of hair loss.

(3) Test on preventing hair loss effect

"noneffective" means a case where no improvement was found in spite of treatment.

"effective" means a case where progress of hair loss was reduced.

"remarkably effective" means a case where hair loss was stopped.

Table 12

| Results of actual use test | | | | |
|---|---|---|---|---|
| Subject | Age | Dandruff | Hair growth | Hair loss |
| 1 | 35 | effective | effective | effective |
| 2 | 45 | effective | remarkably effective | effective |
| 3 | 48 | effective | effective | noneffective |
| 4 | 37 | effective | effective | effective |
| 5 | 35 | noneffective | effective | effective |
| 6 | 26 | effective | noneffective | noneffective |
| 7 | 47 | effective | effective | effective |
| 8 | 46 | remarkably effective | effective | effective |
| 9 | 36 | effective | effective | effective |
| 10 | 31 | effective | effective | effective |
| 11 | 46 | effective | remarkably effective | remarkably effective |
| 12 | 40 | noneffective | effective | |
| 13 | 51 | remarkably effective | effective | remarkably effective |
| 14 | 48 | effective | effective | effective |
| 15 | 25 | effective | noneffective | effective |

Other preparation examples

Besides those described in Examples 25 to 60, compositions for the head of this invention can be provided as those of the following dosage forms.

Example 61

A cream was prepared from Phase A and Phase B of the following compositions.

Composition

| (Phase A) | |
|---|---|
| Liquid paraffin | 10.0 |
| Glyceryl monostearate | 3.0 |
| Vaseline | 5.5 |
| EO(20 mols)-2-octyl dodecyl ether | 3.0 |
| Propyl paraben | 0.3 |
| Perfume | 0.1 |

| (Phase B) | |
|---|---|
| The extract of Example 5 | 1.0 |
| Glycerol | 7.0 |
| Dipropylene glycol | 18.0 |
| Polyethylene glycol 4000 | 5.0 |
| Sodium hexametaphosphate | 0.005 |
| Deionized water | 47.095 |

Preparation

Phase A was heated to give a solution and held at 70°C. Separately, Phase B was heated to give a solution and held at 70°C. Phase B was added into Phase A, the mixture was stirred, and the resultant emulsion was cooled to give a cream-like preparation.

Example 62

Composition (preparation example of lotion)

| | |
|---|---|
| The extract of Example 2 | 0.01 |
| The extract of Example 4 | 0.6 |
| The extract of Example 5 | 0.05 |
| The extract of Example 1 | 0.8 |
| Hardened castor oil-ethylene oxide (40 mols) adduct | 2.0 |
| 95% Ethanol | 55.0 |
| Deionized water | 41.54 |

Example 63

A cream-like preparation was obtained by repeating the same operations as in Example 61 except that Phase A and Phase B of the following compositions were used.

17

| (Phase A) | |
|---|---|
| Liquid paraffin | 10.5 |
| Glyceryl monostearate | 3.0 |
| EO(20 mols)-2-octyl dodecyl ether | 3.0 |
| Propyl paraben | 0.3 |
| Perfume | 0.1 |

| (phase B) | |
|---|---|
| The extract of Example 14 | 1.0 |
| Gycerol | 10.0 |
| Dipropylene glycol | 18.0 |
| Polyethylene glycol 4000 | 5.0 |
| Sodium hexametaphosphate | 0.005 |
| Deionized water | residual part |

Example 64

Composition

| The extract of Example 11 | 0.5 |
|---|---|
| The extract of Example 14 | 0.3 |
| The extract of Example 13 | 0.1 |
| The extract of Example 15 | 0.1 |
| The extract of Example 12 | 0.01 |
| Hardened castor oil-ethylene oxide (40 mols) adduct | 2.0 |
| 95% Ethanol | 54.0 |
| Deionized water | residual part |

Preparation

Deionized water was added to 95% ethanol, hardened castor oil-ethylene oxide (40 mols) adduct was added thereto, the dried extracts (extracts) were added, and the mixture was stirred to give a solution.

Example 65

A preparation of the following composition was obtained using the following dried extracts (extracts) prepared according to Examples 1 to 6.

18

Composition

| | |
|---|---|
| Hinokitiol | 0.1 |
| Swertia japonica extract | 1.0 |
| Vitamin B6 | 0.2 |
| Vitamin E | 0.01 |
| Menthol | 0.2 |
| Salicylic acid | 0.1 |
| Methanol extract of C. gre. (root) | 1.0 |
| Acetone extract of C. gra. (bark) | 0.5 |
| Ethanol extract of C. mer. (root) | 0.4 |
| Ethyl acetate extract of C. ova. (bark) | 0.05 |
| Surface active agent | 0.1 |
| Propylene glycol | 2.0 |
| 75% Ethanol | residual part |

Example 66 (preparation example of shampoo)

Composition

| | |
|---|---|
| Sodium N-cocoyl-N-methyl taurate | 2.0 |
| Polyoxyethylene (8 mols) oleyl alcohol ether | 2.0 |
| Lauryldiethanolamide | 4.0 |
| Ethylene glycol fatty acid ester | 1.0 |
| Glycerol | 0.2 |
| Menthol | 0.1 |
| The extract of Example 20 | 1.0 |
| Disodium edetate | 0.1 |
| Perfume | appropriate amount |
| Purified water | residual part |

Example 67 (Preparation example of rinse)

| | |
|---|---|
| Stearyltrimethylammonium chloride | 1.5 |
| Silicone oil | 3.0 |
| Polyoxyethylene (10 mols) oleyl alcohol ether | 1.0 |
| Glycerol | 5.0 |
| Acetone extract of E. tri. (wood) | 1.0 |
| Acetone extract of E. hel. (part on ground) | 0.3 |
| Methanol extract of E. sup. (bark) | 0.1 |
| Antiseptic | appropriate amount |
| Ultraviolet absorber | appropriate amount |
| Purified water | residual part |

Example 68 (Preparation example of scalp treatment)

| | |
|---|---|
| Liquid paraffin | 27.0 |
| Stearic acid | 5.0 |
| Cetanol | 5.0 |
| Sorbitan monooleate | 2.0 |
| Acetone extract of E. ner. (part on ground) | 1.0 |
| 1,3-Butylene glycol | 5.0 |
| Antiseptic | appropriate amount |
| Purified water | residual part |

Example 69 (Preparation example of hair tonic)

| | |
|---|---|
| Hinokitiol | 0.1 |
| Japanese chirata extract | 1.0 |
| Vitamin B6 | 0.2 |
| Vitamin E | 0.01 |
| Menthol | 0.2 |
| Salicylic acid | 0.1 |
| Ethanol extract of C. gre. (root) | 5.0 |
| Ethyl acetate extract of C. gre. (root) | 0.008 |
| Ethyl acetate extract of C. esc. (leaf) | 0.008 |
| Surface active agent | 0.1 |
| Propylene glycol | 2.0 |
| 75% Ethanol | residual part |

Example 70 (Preparation example of hair tonic)

| | |
|---|---|
| Hinokitiol | 0.1 |
| Japanese chirata extract | 1.0 |
| Vitamin B6 | 0.2 |
| Vitamin E | 0.01 |
| Menthol | 0.2 |
| Salicylic acid | 0.1 |
| The extract of Example 5 | 0.5 |
| The extract of Example 7 | 1.0 |
| The extract of Example 20 | 0.5 |
| Surface active agent | 0.1 |
| Propylene glycol | 2.0 |
| 75% Ethanol | residual part |

Example 71 (Preparation example of rinse)

| | |
|---|---|
| Stearyltrimethylammonium chloride | 1.5 |
| Silicone oil | 3.0 |
| Polyoxyethylene (10 mols) oleyl alcohol ether | 1.0 |
| Glycerol | 5.0 |
| Methanol extract of C. gre. (leaf) | 1.0 |
| Methanol extract of C. gre. (seed) | 2.0 |
| Ethyl acetate extract of C. gre. (bark) | 0.5 |
| Antiseptic | appropriate amount |
| Ultraviolet absorber | appropriate amount |
| Purified water | residual part |

In the actual use test of these preparations (Examples 56 to 64), effects almost equal to the results of the above actual use test on prevention of dandruff and hair loss, and hair growth promotion were attained.

## Claims

1.  A composition comprising an extract derived from a plant, in an amount effective for hair revitalization, hair loss prevention or hair growth induction, and pharmaceutically acceptable auxiliaries, said plant being one or more selected from plants belonging to the family Berberidaceae Juss., the family Flacourtiaceae DC. and the family Euphorbiaceae Juss.

2.  The composition according to claim 1 wherein the plant belonging to the family Berberidaceae Juss. is a plant of the genus Podophyllum L.

3.  The composition according to claim 1 wherein the plant belonging to the family Flacourtiaceae DC. is a plant of the genus Casearia Jacq.

4.  The composition according to claim 1 wherein the plant belonging to the family Euphorbiaceae Juss. is a plant of the genus Euphorbia L.

5.  The composition according to claim 2 wherein the plant belonging to the genus Podophyllum L. is selected from the group consisting of Podophyllum peltatum L., Podophyllum hexandrum Royle, Podophyllum pleianthum Hance, Podophyllum versipella Hance and Podophyllum emodi Wall.

6.  The composition according to claim 3 wherein the plant belonging to the genus Casearia Jacq. is selected from the group consisting of Casearia grewiaefolia Miq., Casearia merrilli Hayatae, Casearia cauliflora Volkens. Casearia esculenta Roxb., Casearia graveolens Daltz. and Casearia ovata Willd.

7.  The composition according to claim 4 wherein the plant belonging to the genus Euphorbia L. is selected from the group consisting of Euphorbia trigona Haw., Euphorbia helioscopia L., Euphorbia lathyris L., Euphorbia neriifolia L., Euphorbia pekinensis Rupr. and Euphorbia supina Rafin.

8.  A method for revitalizing hair, preventing hair loss or inducing hair growth on a mammal, which comprises applying a composition for hair revitalization, hair loss prevention or hair growth induction to the scalp or hair, said composition comprising an effective amount of an extract derived from a plant, said plant being one selected from the group consisting of plants belonging to the family Berberidaceae Juss., the family Flacourtiaceae DC. and the family Euphorbiaceae Juss.

9.  The method according to claim 8 wherein the plant belonging to the family Berberidaceae Juss. is a plant of the genus Podophyllum L.

10. The method according to claim 8 wherein the plant belonging to the family Flacourtiaceae DC. is a plant of the genus Casearia Jacq.

**11.** The method according to claim 8 wherein the plant belonging to the family Euphorbiaceae Juss. is a plant of the genus Euphorbia L.

**12.** The method according to claim 9 wherein the plant belonging to the genus Podophyllum L. is selected from the group consisting of Podophyllum peltatum L., Podophyllum hexandrum Royle, Podophyllum pleianthum Hance, Podophyllum versipella Hance and Podophyllum emodi Wall.

**13.** The method according to claim 10 wherein the plant belonging to the genus Casearia Jacq. is selected from the group consisting of Casearia grewiaefolia Miq., Casearia merrilli Hayatae, Casearia cauliflora Volkens, Casearia esculenta Roxb., Casearia graveolens Daltz. and Casearia ovata Willd.

**14.** The method according to claim 11 wherein the plant belonging to the genus Euphorbia L. is selected from the group consisting of Euphorbia trigona Haw., Euphorbia helioscopia L., Euphorbia lathyris L., Euphorbia neriifolia L., Euphorbia pekinensis Rupr. and Euphorbia supina Rafin.

**15.** Use of an extract derived from a plant for preparing a composition for hair revitalization, hair loss prevention or hair growth induction of a mammal, said plant being one selected from the group consisting of plants belonging to the family Berberidaceae Juss., the family Flacourtiaceae DC. and the family Euphorbiaceae Juss.